# EUROPÄISCHE PATENTSCHRIFT

(11) **EP 1 639 028 B1**
(45) Veröffentlichungstag und Bekanntmachung des Hinweises auf die Patenterteilung: **22.08.2007**
(21) Anmeldenummer: 04729872.4
(22) Anmeldetag: 28.04.2004
(51) Int. Cl.: C08G 63/183

(54) **VERWENDUNG EINES AMORPHEN POLYESTERS ALS POLYMERBASIS FÜR KAUMASSEN**
USE OF AN AMORPHOUS POLYESTER AS A POLYMER BASE FOR MASTICATORY SUBSTANCES
UTILISATION D'UN POLYESTER AMORPHE COMME BASE POLYMERE POUR DES PATES A MACHER

(30) Priorität: 29.04.2003 DE 10319315
(43) Veröffentlichungstag der Anmeldung: 29.03.2006
(73) Patentinhaber: BASF Aktiengesellschaft, 67056 Ludwigshafen (DE)
(72) Erfinder: YAMAMOTO, Motonori, 68199 Mannheim (DE); MACH, Helmut, 69115 Heidelberg (DE); HILLER, Margit, 97753 Karlstadt (DE); RATH, Hans, Peter, 67269 Grünstadt (DE)
(74) Vertreter: Reitstötter - Kinzebach
(86) Internationale Anmeldenummer: PCT/EP2004/004484
(87) Internationale Veröffentlichungsnummer: WO 2004/096886

(56) Entgegenhaltungen:
- DE-A- 4 440 836
- US-B1- 6 444 782

## Beschreibung

Die vorliegende Erfindung betrifft die Verwendung eines amorphen Polyesters als Polymerbasis für Kaumassen. Weiterhin betrifft die Anmeldung Kaumassen, die solche Polyester enthalten.

Übliche Kaumassen (Kaubasen) beruhen auf synthetischen Thermoplasten, wie Polyvinylethylether, Polyvinylisobutylether, Polyisobuten, Isopren-Isobuten-Copolymere (Butyl-Kautschuk), Styrol-Butadlen-Copolymere (SBR-Kautschuk) und Polyvinylacetat (PVA). Nachteilig bei diesen Polymeren ist ihre Klebrigkeit und schlechte biologische Abbaubarkeit. Achtlos entsorgte Kaugummis stellen ein immerwährendes Ärgemis dar, denn einmal auf einer Oberfläche anhaftend lassen sie sich in der Regel nur unter großem Aufwand wieder entfernen. Auch ist ihre Zersetzung durch Umweitfaktoren, wie Regen. Sonnenlicht, mechanischem Abrieb und Abbau durch Mikroorganismen so langsam, dass sich das Problem ihrer Entfernung nicht inhärent löst

Zwar beschreibt die US 6,013,287 eine Kaugummibase, die auf einem Endgruppenverkappten Polyester beruht und nicht sehr klebrig sein soll. Die Alkoholkomponente des Polyesters ist unter Glycerin, Propylenglykol und 1,3-Butandiol ausgewählt und die Säurekomponente unter Fumarsäure, Adipinsäure, Äpfelsäure, Bernsteinsäure und Weinsäure. Die Polysster-Endgruppen sind mit einem monofunktionellen Alkohol oder einer Monocarbonsäure verkappt Nachteillg ist jedoch, dass derartige Polyester unter üblichen Umwelteinflüssen, Insbesondere durch Sonnenlicht, kaum abgebaut werden.

Die EP-A 0711506 beschreibt einen biologisch abbaubaren Kaugummi, der in der Kaubase einen biologisch abbaubaren Polyester oder ein biologisch abbaubares Polycarbonat enthält Der Polyester bzw. das Polycarbonat basieren auf einkondensierten cyclischen Estern bzw. Carbonaten, wie Lactid, Glycolid, δ-Valerotacton, β-Propiolacton, γ-Caprolacton und Trimethylcarbonat. Nachteilig bei solchen Polyestern und Polycarbonaten ist jedoch, dass sie durch UV-Licht kaum abgebaut werden. Außerdem weisen diese Polyester eine geringe Hydrolysestabilität auf, so dass der Kaugummi rasch seine geschmacklichen und taktilen Eigenschaften (Kaugefühl) verliert.

Aufgabe der vorliegenden Erfindung war es daher, eine Polymerbasis für Kaumassen zur Verfügung zu stellen, welche nicht klebrig ist und sowohl biologisch als auch durch UV-Licht abbaubar ist Außerdem sollte die Polymerbasis gleichzeitig eine gute Hydrolysestabilität aufweisen.

Die Aufgabe wird gelöst durch einen amorphen Polyester, welcher als Wiederholungseinheiten
a) wenigstens eine aromatische Dicarbonsäure,
b) wenigstens eine aliphatische Dicarbonsäure und
c) wenigstens ein aliphatisches Diol, das wenigstens eine Verzweigungsstelle, eine gesättigte cyclische Teilstruktur und/oder wenigstens eine Ethergruppe aufweist,

in einkondensierter Form enthält.

Die Erfindung betrifft somit die Verwendung eines derartigen Polyesters als Polymerbasis für Kaumassen sowie Kaumassen, die einen derartigen Polyester enthalten.

Unter "amorph" versteht man im Rahmen der vorliegenden Erfindung solche Polyester, die weniger als 5 Gew.-%, vorzugsweise weniger als 2 Gew.-%, bezogen auf das Gesamtgewicht des Polyesters, kristalline Anteile enthalten. Insbesondere liegt der Anteil an kristallinen Bestandteilen (sofern überhaupt vorhanden) unterhalb üblicher Nachweisgrenzen. Unter kristallinen Bestandteilen versteht man im Rahmen der vorliegenden Erfindung solche, die bei einer DSC-Messung (Differential Scanning Calorimetry; Differentialthermoanalyse) Schmelz- und Kristallisationspeaks aufweisen (endothermer Phasenübergang). Umgekehrt versteht man entsprechend unter amorphen Polyestern solche, die in DSC-Messungen keine messbaren Schmelz- und Kristallisationspeaks aufweisen. Die DSC-Messung zur Bestimmung des amorphen Zustands des Polyesters beruht im Rahmen der vorliegenden Erfindung auf folgendem Verfahren: Verwendet wird ein Gerät der Bezeichnung Exstet DSC 6200R der Fa. Seiko. 10 bis 15 mg der zu untersuchenden Probe wird unter einer Stickstoffatmosphäre mit einer Aufheizrate von 20 °C/min von -100 °C auf 200 °C erwärmt und es wird beobachtet, ob Schmelzpeaks auftreten. Die Probe wird anschließend sofort mit einer Abkühlrate von 20 °C/min von 200 °C auf -100 °C abgekühlt und es wird beobachtet, ob Kristallisationspeaks auftreten. Als Referenz wird ein entsprechender leerer Probentiegel verwendet.

Die erfindungsgemäß verwendeten Polyester sind biologisch abbaubar. Unter biologischer Abbaubarkeit versteht man nach DIN V 54900, dass die Polyester unter Umwelteinflüssen in einer angemessenen und nachweisbaren Zeitspanne zerfallen. Der Abbau kann hydrolytisch und/oder oxidativ erfolgen und wird zum überwiegenden Teil durch die Einwirkung von Mikroorganismen wie Bakterien, Hefen, Pilzen und Algen bewirkt. Die biologische Abbaubarkeit lässt sich z.B. dadurch bestimmen, dass Polyester mit Kompost gemischt und für eine bestimmte Zeit gelagert werden. Gemäß ASTM D 5338, ASTM D 6400, EN 13432 und DIN V 54900 wird CO₂-freie Luft beispielsweise durch gereiften Kompost während des Kompostierens strömen gelassen und dieser einem definierten Temperaturprogramm unterworfen. Hierbei wird die biologische Abbaubarkeit über das Verhältnis der Netto-CO₂-Freisetzung der Probe (nach Abzug der CO₂-Freisetzung durch den Kompost ohne Probe) zur maximalen CO₂-Freisetzung der Probe (berechnet aus dem Kohlenstoffgehalt der Probe) als biologische Abbaubarkeit definiert. Biologisch abbaubare Polyester zeigen in der Regel schon nach wenigen Tagen der Kompostierung deutliche Abbauerscheinungen wie Pilzbewuchs, Riss- und Lochbildung.

Die biologische Abbaubarkeit lässt sich auch dadurch bestimmen, dass man den Polyester mit einer bestimmtem Menge eines geeigneten Enzyms bei einer bestimmtem Temperatur für eine festgesetzte Zeitdauer inkubiert und anschließend die Konzentration der im Inkubationsmedium gelösten organischen Abbauprodukte bestimmt. Beispielsweise kann man analog zu Y. Tokiwa et al., American Chemical Society Symposium 1990, Chapter 12, "Biodegradation of Synthetic Polymers Containing Ester Bonds" den Polyester mit einer vorbestimmten Menge einer Lipase beispielsweise aus Rhizopus arrhizus, Rhizopus delemar, Achromobacter sp. oder Candida cylindracea bei 30 bis 37°C mehrere Stunden inkubieren, und anschließend den DOC-Wert (dissolved organic carbon) des von unlöslichen Bestandteilen befreiten Reaktionsgemischs messen. Als biologisch abbaubar gelten im Rahmen der vorliegenden Erfindung solche Polyester, die nach der enzymatischen Behandlung mit einer Lipase aus Rhizopus arrhizus bei 35°C nach 16 h einen DOC-Wert ergeben, der wenigstens 10 Mal höher ist als derjenige desselben Polyesters, der nicht mit dem Enzym behandelt wurde.

Die erfindungsgemäß verwendeten Polyester werden insbesondere auch durch UV-Licht, d.h. durch Sonnenlicht, zersetzt, d.h. die Polyester zerfallen in einer angemessenen und nachweisbaren Zeitspanne, wobei der Abbau im Wesentlichen durch Sonnenlicht bewirkt wird. Die UV-Abbaubarkeit lässt sich z. B. dadurch bestimmen, dass man den Polyester mit künstlichem UV-Licht einer bestimmtem Strahlungsstärke für eine bestimmte Zeitdauer bestrahlt und die Veränderungen des Polyesters misst Beispielsweise werden die Polyester mit einer Wellenlänge von 300 bis 800 nm und einer Leistung von 765 W/m² 8 Wochen bestrahlt und ihre Viskositätszahl wird regelmäßig, z.B. jede Woche, bestimmt. Durch UV-Licht abbaubare Polyester zeigen in der Regel schon nach wenigen Tagen deutliche Veränderungen, insbesondere eine deutliche Abnahme der Viskositätszahl. Als durch UV-Licht abbaubar gelten im Rahmen der vorliegenden Erfindung Polyester, deren Viskositätszahl nach 3 Wochen Bestrahlung um wenigstens 50 % abnimmt.

Die aromatische Dicarbonsäure a) enthält zwei Carboxylgruppen, die an ein aromatisches System gebunden sind. Vorzugsweise handelt es sich bei dem aromatischen System einen Carboaromaten, wie Phenyl oder Naphthyl. Bei mehrkernigen Aromaten können die beiden Carboxylgruppen an den gleichen oder an unterschiedliche Ringe gebunden sein. Das aromatische System kann auch eine oder mehrere Alkylgruppen, z. B. Methylgruppen, aufweisen. Die aromatische Dicarbonsäure ist in der Regel unter aromatischen Dicarbonsäuren mit 8 bis 12 Kohlenstoffatomen, wie Phthalsäure, 1-sophthalsäure, Terephthalsäure, 1,5- und 2,6-Naphthalindicarbonsäure ausgewählt. Bevorzugte aromatische Dicarbonsäuren sind Terephthalsäure, Isophthalsäure und Phthalsäure sowie Gemische davon. Insbesondere handelt es sich bei der aromatischen Dicarbonsäure um Terephthalsäure oder um ein Gemisch aromatischer Dicarbonsäuren, welches wenigstens 80 Gew.-%, vorzugsweise wenigstens 90 Gew.-% und insbesondere wenigstens 95 Gew.-%, bezogen auf das Gesamtgewicht des Gemischs, Terephthalsäure und wenigstens eine der vorstehend genannten aromatischen C₈-C₁₂-Dicarbonsäuren enthält.

Die aliphatische Dicarbonsäure b) ist in der Regel unter aliphatischen Dicarbonsäuren mit 4 bis 12 Kohlenstoffatomen, wie Bernsteinsäure, Glutarsäure, 2-Methylglutarsäure, 3-Methylglutarsäure, 2,2-Dimethylglutarsäure, Adipinsäure, Pimelinsäure, Suberinsäure, Azelainsäure und Sebacinsäure, höheren Homologen und Stereoisomeren sowie Gemischen davon ausgewählt. Bevorzugte aliphatische Dicarbonsäuren sind Bernsteinsäure, Glutarsäure, Adipinsäure, Pimelinsäure, Azelainsäure und Sebacinsäure sowie Gemische davon. Insbesondere handelt es sich bei der aliphatischen C₄-C₁₂-Dicarbonsäure um Adipinsäure oder um ein Gemisch aliphatischer Dicarbonsäuren, welches wenigstens 80 Gew.-%, vorzugsweise wenigstens 90 Gew.-% und insbesondere wenigstens 95 Gew.-%, bezogen auf das Gesamtgewicht des Gemischs, Adipinsäure und wenigstens eine der vorstehend genannten aliphatischen C₄-C₁₂-Dicarbonsäuren enthält.

Das Molverhältnis von aromatischer Dicarbonsäure a) zu aliphatischer Dicarbonsäure b) beträgt 1:4 bis 2:1, besonders bevorzugt 1:2 bis 3:2 und insbesondere 2:3 bis 1:1.

Als aliphatische Diole c) kommen grundsätzlich verzweigte aliphatische Diole, solche mit einer gesättigten cyclischen Teilstruktur und/oder wenigstens einer Ethergruppe in Betracht. Das aliphatische Diol ist vorzugsweise unter 2,2-Dimethylpropan-1,3-diol (Neopentylglycol), Diethylenglycol, Triethylenglycol, Tetraethylenglycol, Pentaethylenglycol, Hexaethylenglycol, Cyclohexandimethanol sowie Gemischen davon und besonders bevorzugt unter Neopentylglycol, Diethylenglycol, Triethylenglycol und Gemischen davon ausgewählt.

Außerdem kann der Polyester bis zu 20 Gew.-%, vorzugsweise bis zu 10 Gew.-% und insbesondere bis zu 5 Gew.-% eines von c) verschiedenen Diols in einkondensierter Form enthalten. Beispiele für geeignete Diole sind unverzweigte aliphatische C₂-C₁₂-Diole, wie Ethylenglykol, 1,3-Propandiol. 1,4-Butandiol, 1,5-Pentandiol, 1,6-Hexandiol und dergleichen. Die Gew.-%-Angaben beziehen sich auf die Gesamtmenge an Diolen c) und den vorstehend genannten, von c) verschiedenen Diolen. Vorzugsweise enthält der Polyester jedoch kein solches Diol.

Vorzugsweise enthält der erfindungsgemäß verwendete Polyester als Wiederholungseinheit weiterhin wenigstens eine Verbindung d) mit wenigstens drei zur Esterbildung befähigten Gruppen in einkondensierter Form.

Solche Verbindungen d), die auch als Verzweiger bezeichnet werden, enthalten vorzugsweise 3 bis 10 funktionelle Gruppen, besonders bevorzugt 3 bis 6 funktionelle Gruppen, die zur Ausbildung von Esterbindungen befähigt sind. Insbesondere handelt es sich dabei um Hydroxygruppen und Carboxylgruppen. Besonders bevorzugte Verzweiger d) enthalten daher 3 bis 6 Hydroxygruppen und/oder Carboxylgruppen.

Bevorzugt sind dabei diese Verbindungen ausgewählt unter Weinsäure, Zitronensäure. Äpfelsäure, Trimethylolpropan, Trimethylolethan, Pentaerythrit, Polyethertriole, Glycerin, Trimesinsäure, Trimellitsäure, Pyromellitsäure und Hydroxyisophthalsäure. Ein besonders bevorzugter Verzweiger d) ist Glycerin.

Der erfindungsgemäß verwendete Polyester enthält den Verzweiger d) in einer Menge von vorzugsweise 0,1 bis 5 Gew.-%, besonders bevorzugt von 0,5 bis 3 Gew.-% und insbesondere von 1 bis 1,5 Gew.-%, bezogen auf das Gesamtgewicht der Polyester-bildenden Bestandteile.

Weiterhin kann der erfindungsgemäß verwendete Polyester einen oder mehrere Kettenverlängerer in einkondensierter Form enthalten. Geeignete Kettenverlängerer sind insbesondere Isocyanate, Divinylether und Bisoxazoline.

Geeignete Isocyanate sind aromatische oder aliphatische Diisocyanate sowie höher funktionelle Isocyanate. Beispiele für geeignete Isocyanate sind
- aromatische Diisocyanate, wie Toluylen-2,4-düsocyanat, Toluylen-2,6-diisocyanat, 2,2'-Diphenylmethandiisocyanat, 2.4'-Diphenytmethandiisocyanat, 4,4'-Diphenylmethandiisocyanat, Naphthylen-1,5-diisocyanat und Xylylen-diisocyanat, wobei 2.2'-, 2,4'- und 4,4'-Diphenylmethandiisocyanat bevorzugt sind. Geeignet sind auch Gemische dieser Diisocyanate. Ein bevorzugtes höher funktionelles Isocyanat ist das dreikernige aromatische Triisocyanat Tri(4-isocyanatophenyl)methan. Die mehrkernigen aromatischen Isocyanate fallen beispielsweise bei der Herstellung von 1- oder 2-kernigen Diisocyanaten an.
- aliphatische Diisocyanate, insbesondere lineare oder verzweigte Alkylendiisocyanate oder Cycloalkylendiisocyanate mit 2 bis 20, vorzugsweise mit 3 bis 12 Kohlenstoffatomen, wie 1,6-Hexamethylendiisocyanat, Isophorondiisocyanat und Methylen-bis(4-isocyanatocyclohexan). Bevorzugt sind dabei 1,6- Hexamethylendiisocyanat und 1-sophorondiisocyanat.
- Isocyanurate, insbesondere aliphatische lsocyanurate, die sich von. Alkylendiisocyanaten oder Cydoaikytendiisocyanaten mit 2 bis 20 Kohlenstoffatomen, vorzugsweise mit 3 bis 12 Kohlenstoffatomen, wie Isophorondiisocyanat oder Methylen-bis(4-isocyanatocyclohexan) ableiten, z. B. lsocyanurate, die sich von n-Hexamethylendiisocyanat ableiten, insbesondere cyclische Trimere, Pentamere oder höhere Oligomere des n-Hexamethylendlisocyanats.

Geeignete Divinylether sind alle üblichen und kommerziell erhältlichen Divinylether. Bevorzugte Divinylether sind 1,4-Butandioldivinylether, 1,6-Hexandioldivinylether und 1,4-Cyclohexandiinethanoldivinylether oder Gemische davon.

Geeignete Bisoxazoline sind 2,2'-Bisoxazoline der Formel 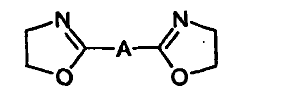 worin A für eine Einfachbindung, eine C₂-C₄-Alkylenbrücke, wie 1,2-Ethylen, 1,2- oder 1,3-Propylen, 1,2-,1,3-,1,4- oder 2,3-Butylen, oder für Phenylen steht, in einkondensierter Form enthalten.

Die Bisoxazoline sind beispielsweise durch das in Angew. Chem. Int. Ed., Band 11 (1972), S. 287-288 beschriebene Verfahren erhältlich.

Bevorzugte Bisoxazoline sind 2,2'-Bis(2-oxazolin), Bis(2-oxazolinyl)methan, 1,2-Bis(2-oxazolinyl)ethan, 1,3-Bis(2-oxazolinyl)propan, 1,4-Bis(2-oxazolinyl)butan, 1,4-Bis(2-oxazolinyl)benzol, 1,3-Bis(2-oxazolinyl)benzol und 1,2-Bis(2-oxazolinyl)benzol.

Wenn der erfindungsgemäß verwendete Polyester solche Kettenverlängerer enthält, so sind diese in einer Menge von vorzugsweise 0,01 bis 5 Gew.-%, besonders bevorzugt von 0,05 bis 4 Gew.-%, bezogen auf das Gesamtgewicht der Polyester-bildenden Bestandteile a), b) und c), enthalten.

Vorzugsweise enthält der Polyester keine, d.h. weniger als 0,1 Gew.-%, bezogen auf das Gesamtgewicht des Polyesters, Kettenverlängerer.

Vorzugsweise ist der erfindungsgemäß verwendete Polyester zu wenigstens 95 Gew.-%, besonders bevorzugt zu wenigstens 96 Gew.-% und insbesondere zu wenigstens 98 Gew.-%, beispielsweise zu 98 bis 99,9 Gew.-%, aus den Komponenten a), b), c) und d) aufgebaut.

Der erfindungsgemäß verwendete Polyester weist vorzugsweise eine Glasübergangstemperatur T_{g} von -60 bis 0 °C, besonders bevorzugt von -50 °C bis 0 °C auf. Die angegebenen T_{g}-Werte wurden durch DSC-Messungen bestimmt. Die DSC-Messungen erfolgten nach üblichen Verfahren des Standes der Technik, die dem Fachmann bekannt sind.

Außerdem ist der erfindungsgemäß verwendete Polyester charakterisiert durch eine Viskositätszahl im Bereich von in der Regel 30 bis 250 ml/g, vorzugsweise 50 bis 200 ml/g und insbesondere 80 bis 150 ml/g (gemessen in o-Dichlorbenzol/Phenol (Gewichtsverhältnis 50:50) bei einer Konzentration von 0,5 Gew.-% Polyester bei einer Temperatur von 25 °C gemäß EN ISO 1628-1).

Die Herstellung des erfindungsgemäß einzusetzenden Polyesters erfolgt nach an sich bekannten Verfahren, wie sie beispielsweise in Sorensen und Campbell, "Preparative Methods of Polymer Chemistry", Interscience Publishers, Inc., New York, 1961, Seiten 111 bis 127; Encycl., of Polym. Science and Eng., Band 12, 2. Auflage, John Wiley & Sons, 1988, Seiten 1 bis 75; Kunststoff-Handbuch, Band 3/1, Carl Hanser Verlag, München, 1992, Seiten 15 bis 32; WO 92/13019; EP-A 568593; EP-A 565235; EP-A 28687; EP-A 792309 und EP-A 792310 beschrieben sind, auf die hiermit in vollem Umfang Bezug genommen wird.

In das Herstellungsverfahren können die Dicarbonsäuren a) und b) entweder in Form der Säure oder als Ester-bildende Derivate eingesetzt werden. Ester-bildende Derivate sind beispielsweise die Anhydride dieser Säuren oder deren Ester, beispielsweise mit C₁-C₆-Alkanolen, wie Methanol, Ethanol, n-Propanol, Isopropanol, n-Butanol, sec-Butanol, Isobutanol, tert-Butanol, n-Pentanol, Isopentanol oder n-Hexanol. Entsprechendes gilt für Komponente d), wenn diese Carboxylgruppen enthält.

In Abhängigkeit davon, ob ein Überschuss an Säure- oder Hydroxy-Endgruppen gewünscht ist, können entweder die Säurekomponenten a) bzw. b) oder die Diolkomponente c) im Überschuss eingesetzt werden. Vorzugsweise werden jedoch die Säurekomponenten a) und b) und die Diolkomponente c) in einem Molverhältnis von [a) + b)]:c) = 1:1 bis 1:2,5, besonders bevorzugt 1:1,1 bis 1:1,5 eingesetzt.

Nur als Beispiel sei die Umsetzung der Polyester-bildenden Bestandteile zunächst bei Temperaturen im Bereich von 160 bis 230 °C in der Schmelze bei Atmosphärendruck, vorzugsweise unter Inertgasatmosphäre, und anschließend zur Vervollständigung der Polykondensation bis zum gewünschten Molekulargewicht bei einer Temperatur von 180 bis 260 °C und unter verringertem Druck genannt (siehe Tsal et al., Polymer 1990, 31, 1589).

Ein weiterer Gegenstand der vorliegenden Erfindung ist eine Kaumasse, enthaltend wenigstens einen wie vorstehend definierten Polyester sowie wenigstens einen weiteren Zusatzstoff.

Als Kaumasse (Kaubase) bezeichnet man im Allgemeinen den wasserunlöslichen, unverdaulichen, beim Kauen plastisch werdenden Bestandteil von Kaugummis (siehe Römpp Chemie-Lexikon, 9. Auflage, Georg Thieme Verlag, Stuttgart, New York, S. 2181). Üblicherweise enthält die Kaumasse neben der polymeren Basis weitere Zusatzstoffe, wie Harze, Wachse, Fette und Öle, die in der Regel als Weichmacher und Emulgatoren dienen und die taktilen Eigenschaften (Kaubarkeit, Mundgefühl) verbessern, ferner anorganische Füllstoffe, Farbmittel, Bleichmittel und Antioxidantien.

Vorzugsweise enthält die Kaumasse den Polyester in einer Menge von 20 bis 90 Gew.-%, besonders bevorzugt von 20 bis 70 Gew.-% und insbesondere von 20 bis 50 Gew.-%, bezogen auf das Gesamtgewicht der Kaumasse.

Geeignete Harze sind beispielsweise Kolophonium-Derivate, wie Pentaerythrit-Ester von Kolophonium, hydriertem oder teilhydriertem Kolophonium und Glycerin-Ester von Kolophonium, hydriertem, teilhydriertem, teildimerisiertem oder polymerisiertem Kolophonium, ferner Terpen-Harze, wie polymerisiertes α- oder β-Pinen. Wenn die Kaumasse Harze enthält, so sind diese in der Regel in einer Menge von 5 bis 30 Gew.-%, bezogen auf das Gesamtgewicht der Kaumasse, enthalten.

Geeignete Wachse sind beispielsweise pflanzliche Wachse, wie Candelillawachs und Carnaubawachs, tierische Wachse, wie Bienenwachs und Lanolin, und petrochemische Wachse, wie Paraffinwachse und Mikrowachse (mikrokristalline Wachse). Wenn die Kaumasse Wachse enthält, so sind diese in der Regel in einer Menge von 1 bis 15 Gew.-%. bezogen auf das Gesamtgewicht der Kaumasse, enthalten.

Geeignete Fette und Öle sind beispielsweise Talg, hydrierter Talg, hydrierte und teil-hydrierte pflanzliche Öle, wie Sojaöl, Sonnenblumenöl, Maisöl, Rapsöl, Erdnussöl, Palmöl und Baumwollsamenöl, Kakaobutter, Glycerinmonostearat, Glycerintriacetat, Lecithin, Fettsäuremono-, -di- und -triglyceride, acetylierte Monoglyceride, Fettsäuren, wie Stearinsäure, Palmitinsäure, Ölsäure und Linolsäure, sowie deren Gemische. Wenn die Kaumasse Fette und Öle enthält, so sind diese in der Regel in einer Menge von 5 bis 30 Gew.-%, bezogen auf das Gesamtgewicht der Kaumasse, enthalten.

Geeignete Füllstoffe sind beispielsweise Magnesium- und Calciumcarbonat, gemahlener Kalkstein, Talk, Silikate, wie Magnesium- und Aluminiumsilikate, Ton, Aluminiumoxid, Titanoxid, Mono-, Di- und Tricalciumphosphat, Cellulosepolymere und deren Gemische. Wenn die Kaumasse Füllstoffe enthält, so sind diese in der Regel in einer Menge von 5 bis 30 Gew.-%, bezogen auf das Gesamtgewicht der Kaumasse, enthalten.

Der Begriff "Farbmittel" umfasst hier und im Folgenden natürliche Farbstoffe, naturidentische Farbstoffe und synthetische Farbstoffe sowie Pigmente. Geeignete Farbmittel und Bleichmittel sind insbesondere solche, die lebensmittelgeeignet sind, beispielsweise Frucht- und Gemüseextrakte, Titandioxid und deren Gemische. Wenn die Kaumasse Farbmittel und Bleichmittel enthält, so sind diese in der Regel in einer Menge von 0,01 bis 1 Gew.-%, bezogen auf das Gesamtgewicht der Kaumasse, enthalten.

Geeignete Antioxidantien sind solche, die lebensmittelgeeignet sind, beispielsweise Butylhydroxyanisol. Butylhydroxytoluol und Propylgallat. Wenn die Kaumasse Antioxidantien enthält, so sind diese in der Regel in einer Menge von 0,01 bis 1 Gew.-%, bezogen auf das Gesamtgewicht der Kaumasse, enthalten.

Außerdem kann die Kaumasse natürliche Elastomere, wie Chicle, Jelutong, Lechi caspi, Gutta Hang Kang, Gutta Soh, Gutta Siak, Massaranduba balata, Massaranduba chocolate und dergleichen enthalten. Wenn die Kaumasse natürliche Elastomere enthält, so sind diese in der Regel in einer Menge von 1 bis 30 Gew.-%, bezogen auf das Gesamtgewicht der Kaumasse, enthalten.

In einer speziellen Ausführungsform enthält die Kaumasse keine Bestandteile tierischen Ursprungs, insbesondere keine tierischen Wachse, Fette und Öle, so dass sie den Anforderungen an koschere Lebensmittel genügt

Die erfindungsgemäße Kaumasse ist nach üblichen Verfahren des Standes der Technik erhältlich, beispielsweise durch inniges Vermischen der Bestandteile.

Schließlich ist Gegenstand der vorliegenden Erfindung ein Kaugummi, enthaltend eine wie oben definierte Kaumasse sowie weitere Zusatzkomponenten, insbesondere wenigstens ein Süßungsmittel und wenigstens einen Geschmacksstoff.

Üblicherweise besteht ein Kaugummi aus einer wasserunlöslichen Kaumasse, einem wasserlöslichen Anteil und Geschmackstoffen (siehe US 6,013.287 und EP-A 0711506).

Der wasserlösliche Bestandteil umfasst im Allgemeinen Weichmacher und Süßungsmittel. Die Weichmacher werden dem Kaugummi zugesetzt, um die Kaubarkeit und das Mundgefühl des Kaugummis zu verbessern.

Beispiele für geeignete Weichmacher sind Glycerin, Lecithin und deren Gemische. Als Weichmacher und Emulgatoren können ferner Sorbitol, hydrierte Stärkehydrolysate, Maissirup sowie deren Gemische verwendet werden.

Süßungsmittel umfassen sowohl Zucker als auch Zuckeraustauschstoffe und Süßstoffe.

Geeignete Zucker sind beispielsweise Saccharose, Dextrose, Maltose, Dextrin, Invertzucker, Glucose, Fructose, Galactose und dergleichen sowie deren Gemische.

Beispiele für geeignete Zuckeraustauschstoffe sind Zuckeralkohole, wie Sorbit, Mannit, Isomalt (Palatinit), Xylit, hydrierte Stärkehydrolysate, Maltit, Lactit und dergleichen sowie deren Gemische.

Als (künstliche) Süßstoffe sind beispielsweise Sucralose, Aspartam, Acesulfam-Salze, Alitam, Saccharin und dessen Salze, Cyclamate, Glycyrrhizin, Dihydrochalcone, Thaumatin, Monellin, Dulcin, Steviosid und dergleichen zu nennen.

Geeignete Geschmacksstoffe sind in der Regel wasserunlöslich und umfassen Pflanzen- und Fruchtöle, wie Zitrusöl, Fruchtessenzen, Pfefferminzöl, Krauseminzeöl (Spearmint), andere Minzöle, Nelkenöl, Anisöl und dergleichen. Auch künstliche Geschmackstoffe können verwendet werden.

Vorzugsweise ist die Kaumasse im Kaugummi in einer Menge von 5 bis 95 Gew.-%, besonders bevorzugt von 10 bis 50 Gew.-% und insbesondere von 20 bis 35 Gew.-%, bezogen auf das Gesamtgewicht des Kaugummis, enthalten.

Der erfindungsgemäße Kaugummi enthält die wasserlöslichen Bestandteile in einer Menge von vorzugsweise 3 bis 94,9 Gew.-%, besonders bevorzugt von 49 bis 89,5 Gew.%, bezogen auf das Gesamtgewicht des Kaugummis.

Die Geschmackstoffe sind in einer Menge von vorzugsweise 0,1 bis 2 Gew.-%, besonders bevorzugt von 0,5 bis 1 Gew.-%, bezogen auf das Gesamtgewicht des Kaugummis, im erfindungsgemäßen Kaugummi enthalten.

In einer speziellen Ausführungsform enthält der Kaugummi keine Bestandteile tierischen Ursprungs, insbesondere keine tierischen Wachse, Fette und Öle, so dass er den Anforderungen an koschere Lebensmittel genügt.

Der erfindungsgemäße Kaugummi ist nach üblichen Verfahren des Standes der Technik erhältlich, beispielsweise durch inniges Vermischen der Bestandteile.

Kaumassen und Kaugummis, die den vorstehend beschriebenen amorphen Polyester als Polymerbasis enthalten, sind auch auf raueren Oberflächen, wie Beton, praktisch nicht klebrig, besitzen eine gute Hydrolysestabilität und werden biologisch und durch Sonnenlicht gut abgebaut.

Die nachfolgenden Beispiele sollen die Erfindung verdeutlichen, ohne sie jedoch einzuschränken.

### Beispiele

### 1. Herstellung der Polyester

### 1.1

746 g Terephthalsäure (4,5 mol), 803 g Adipinsäure (5,5 mol), 1272 g (12 mol) Diethylenglykol und 33,7 g (0,37 mol) Glycerin wurden nach dem Schmelzkondensationsverfahren von Tsai et al., Polymer, 31, 1589 (1990) polycokondensiert. Der erhaltene Polyester besaß eine Viskositätszahl von 125 ml/g (Bestimmung wie vorstehend beschrieben).

### 1.2

747 g (4,5 mol) Terephthalsäure, 803 g (5,5 mol) Adipinsäure , 1248 g (12 mol) Neopentylglykol und 33,8 g (0,37 mol) Glycerin wurden ebenfalls nach dem Schmelzkondensationsverfahren von Tsai et al., Polymer, 31, 1589 (1990) polycokondensiert. Der erhaltene Polyester besaß eine Viskositätszahl von 132 ml/g (Bestimmung wie vorstehend beschrieben).

### Vergleichsbeispiel 1

25 Mol-% L-Lactid, 25 Mol-% D-Lactid und 50 Mol-% ε-Caprolacton wurden gemäß EP-A 0711 506, Beispiel 3, polycokondensiert. Der erhaltene Polyester besaß eine Viskositätszahl von 100 ml/g (Bestimmung wie vorstehend beschrieben).

### 2. Anwendungsbeispiele

Die Polyester aus den Beispielen 1 und 2 und aus dem Vergleichsbeispiel 1 sowie als Vergleichsbeispiel 2 ein mittelmolekulares Polyisobuten wurden auf ihre Entfembarkeit, Hydrolysestabilität, UV-Abbaubarkeit und biologische Abbaubarkeit hin untersucht.

### 2.1 Entfernbarkeit

10 g einer Polymerplatte (5 x 7 cm²) wurde bei Raumtemperatur auf Betonboden gedrückt und per Hand entfernt. Das Gewicht des entfernten Polymers wurde bestimmt. Dabei wurden die gemessenen Polymergewichte wie folgt bewertet.

| Gewicht des entfernten Polymers [g] | Note |
|---|---|
| 8-10 | 1 |
| 6-7,9 | 2 |
| 4-5,9 | 3 |
| 0-3,9 | 4 |

Die Entfernbarkeit der Polymere aus den Beispielen 1 und 2 und den Vergleichsbeispielen 1 und 2 wurde wie in nachfolgender Tabelle 1 aufgeführt bewertet:

**Tabelle 1**

| Polymer | Note |
|---|---|
| Beispiel 1 | 1 (9,5 g) |
| Beispiel 2 | 1 (10 g) |
| Vergleichsbeispiel 1 | 2 (7 g) |
| Vergleichsbeispiel 2 | 3 (5 g) |

Wie die vorstehende Tabelle zeigt, lassen sich die erfindungsgemäß verwendeten Polyester wesentlich besser vom Betonboden entfernen als Polymere des Standes der Technik.

### 2.2 Hydrolysestabilität

Die oben genannten Polymere wurden in Wasser bei 30 °C gelagert und die Abnahme der Viskositätszahl wurde nach 2 bzw. 4 Wochen gemessen.

Die Ergebnisse sind in nachfolgender Tabelle 2 aufgeführt.

**Tabelle 2**

| Polymer | VZ* (Anfang) | VZ* (nach 2 Wochen) | VZ* (nach 4 Wochen) |
|---|---|---|---|
| Beispiel 1 | 125 | 113 | 108 |
| Beispiel 2 | 132 | 125 | 115 |
| Vergleichsbeispiel 1 | 100 | 60 | 35 |

| | | | |
|---|---|---|---|
| * Viskositätszahl [ml/g] | | | |

Wie die obige Tabelle zeigt, sind die erfindungsgemäß verwendeten Polyester wesentlich hydrolysestabiler als der Polyester des Standes der Technik.

### 2.3 UV-Abbaubarkeit

Zur Bestimmung der UV-Abbaubarkeit wurde ein Schnellbelichtungsgerät "SUNTEST" der Firma Heräus verwendet. Bestrahlt wurde mit einer Wellenlänge von 300 bis 800 nm und einer Leistung von 765 W/m². Die Polymere wurden unter diesen Bedingungen insgesamt 8 Wochen lang belichtet, wobei die Viskositätszahl der Polymere nach jeweils nach einer, zwei, drei, vier, sechs und acht Wochen bestimmt wurde. Die Ergebnisse sind in nachfolgender Tabelle 3 aufgeführt.

**Tabelle 3**

| Polymer | VZ* (Anfang) | VZ* (1 Woche) | VZ* (2 Wochen) | VZ* (3 Wochen) | VZ* (4 Wochen) | VZ* (6 Wochen) | VZ* (8 Wochen) |
|---|---|---|---|---|---|---|---|
| Beispiel 1 (ohne Belichtung) | 125 | 124 | 125 | 121 | 122 | 121 | 121 |
| Beispiel 1 | 125 | 74 | 59 | 50 | 46 | 35 | 18 |
| Beispiel 2 | 132 | 87 | 67 | 51 | 43 | 36 | 15 |
| Vergleichsbeispiel 1 | 100 | 91 | 87 | 84 | 79 | 73 | 69 |

| | | | | | | | |
|---|---|---|---|---|---|---|---|
| * Viskositätszahl [ml/g] | | | | | | | |

Wie die obige Tabelle zeigt, sind die erfindungsgemäß verwendeten Polyester wesentlich besser durch UV-Licht abbaubar als der Polyester des Standes der Technik.

### 3.4 Biologische Abbaubarkeit

Zur Bestimmung der biologischen Abbaubarkeit gab man jeweils 30 mg des amorphen Polymers. 2 ml Kaliumhydrogenphosphatpuffer (20 mM, pH 7,0) und 100 Units (1 Unit entspricht der Enzymmenge, die ein µmol Ölsäure pro Minute freisetzt) Lipase aus Rhizopus arrhizus der Firma Sigma in 2 ml Eppendorf-Reaktionsgefäße. Das Reaktionsgemisch wurde bei 35 °C 16 Stunden auf einem Schwenker inkubiert. Nach der Inkubation wurde die Reaktionsmischung zentrifugiert und der DOC-Wert (dissolved organic carbon) des Überstands gemessen. Zur DOC-Messung wurde ein Shimadzu DOC-Analyser verwendet. Analog dazu wurde je eine DOC-Messung nur mit Puffer und Enzym (Enzymkontrolle) und eine nur mit Puffer und Polymer (Blindwert) durchgeführt. Die Ergebnisse sind in nachfolgender Tabelle 4 aufgeführt.

**Tabelle 4**

| Polymer | DOC [mg/l] |
|---|---|
| Beispiel 1 | 567 |
| Beispiel 1 ohne Enzym | 23 |
| Beispiel 2 | 456 |
| Beispiel 2 ohne Enzym | 13 |
| Vergleichsbeispiel 2 | 13 |
| Vergleichsbeispiel 2 ohne Enzym | 11 |
| Puffer | 5 |
| Puffer ohne Enzym | 3 |

Wie die obige Tabelle zeigt, werden die erfindungsgemäß verwendeten Polyester sehr gut biologisch abgebaut, während das in konventionellen Kaumassen verwendete Polyisobuten kaum zersetzt wird.

### 3. Kaubasen

Die in Tabelle 5 angegebenen Bestandteile wurden in den angegebenen Gewichtsverhältnissen innig miteinander zu einer Kaubase vermischt. Hierzu wurde in einem Kneter zunächst der Polyester auf 140 °C unter Kneten erwärmt und anschließend wurden sukzessive der Füllstoff, das Harz, das Fett und das Wachs zugegeben und die Bestandteile zu einer einheitlichen Masse verknetet.

**Tabelle 5**

| Kaubase Nr. | 1 | 2 | 3 | 4 | 5 | 6 | 7 |
|---|---|---|---|---|---|---|---|
| Polyester [Gew.-%] | 30 | 39,5 | 38 | 43 | 40 | 28 | 35 |
| Füllstoff [Gew.-%] | 25 | 25 | 23 | 23 | 22 | 27 | 21 |
| Wachs [Gew.-%] | 10 | 5 | 5 | 4 | 8 | 5 | 5 |
| Harz [Gew.-%] | 20 | 20 | 20 | 10 | 20 | 20 | 20 |
| Fett [Gew.-%] | 15 | 10,5 | 14 | 20 | 10 | 20 | 19 |

Als Polyester wurden die Polyester der Beispiele 1 und 2 eingesetzt. Als Füllstoff verwendete man Kalkstein. Als Wachs wurde mikrokristallines Wachs eingesetzt. Bei den verwendeten Harzen handelte es sich um Kolophoniumderivate der Bezeichnung Picolite C 115 und MBG 429 der Firma Eastman. Als Fett wurde hydriertes oder teilhydriertes pflanzliches Öl bzw. Glycerinmonostearat eingesetzt.

## Patentansprüche

1. Verwendung eines amorphen Polyesters, welcher als Wiederholungseinheiten
a) wenigstens eine aromatische Dicarbonsäure,
b) wenigstens eine aliphatische Dicarbonsäure und
c) wenigstens ein aliphatisches Diol, das wenigstens eine Verzweigungsstelle, eine gesättigte cyclische Teilstruktur und/oder wenigstens eine Ethergruppe aufweist,
in einkondensierter Form enthält, als Polymerbasis für Kaumassen, wobei das Molverhältnis von a) zu b) 1:4 bis 2:1 beträgt.

2. Verwendung nach Anspruch 1, wobei die Glasübergangstemperatur T_{g} des Polyesters 0 bis -60 °C beträgt.

3. Verwendung nach einem der vorhergehenden Ansprüche, wobei die aromatische Dicarbonsäure ausgewählt ist unter Terephthalsäure, Isophthalsäure und Phthalsäure.

4. Verwendung nach einem der vorhergehenden Ansprüche, wobei die aliphatische Dicarbonsäure ausgewählt ist unter C₄-C₁₂-Dicarbonsäuren.

5. Verwendung nach Anspruch 4, wobei die aliphatische C₄-C₁₂-Dicarbonsäure ausgewählt ist unter Bernsteinsäure, Glutarsäure, Adipinsäure, Pimelinsäure, Azelainsäure und Sebacinsäure.

6. Verwendung nach einem der vorhergehenden Ansprüche, wobei das aliphatische Diol ausgewählt ist unter 2,2-Dimethylpropan-1,3-diol, Diethylenglykol, Triethylenglykol, Tetraethylenglykol, Pentaethylenglykol, Hexaethylenglykol und Cyclohexandimethanol.

7. Verwendung nach einem der vorhergehenden Ansprüche, wobei der Polyester als Wiederholungseinheit weiterhin
d) wenigstens eine Verbindung mit wenigstens drei zur Esterbildung befähigten Gruppen
in einkondensierter Form enthält.

8. Verwendung nach Anspruch 7. wobei die Verbindung mit wenigstens drei zur Esterbildung befähigten Gruppen ausgewählt ist unter Weinsäure, Zitronensäure, Äpfelsäure, Trimethylolpropan, Trimethylolethan, Pentaerythrit, Polyethertriolen, Glycerin, Trimesinsäure, Trimellitsäure, Pyromellitsäure und Hydroxyisophthalsäure.

9. Verwendung nach einem der Ansprüche 7 oder 8, wobei der Polyester die Komponente d) in einer Menge von 0,1 bis 5 Gew.-%, bezogen auf das Gesamtgewicht der Polyester-bildenden Bestandteile, in einkondensierter Form enthält.

10. Kaugummi, enthaltend eine Kaumasse, die wenigstens einen wie in einem der vorhergehenden Ansprüchen definierten, amorphen Polyester sowie wenigstens einen weiteren, unter Harzen, Wachsen, Fetten und Ölen ausgewählten Zusatzstoff umfasst, wenigstens ein Süßungsmittel und wenigstens einen Geschmacksstoff.

## Claims

1. The use of an amorphous polyester which comprises as repeating units in condensed form
a) at least one aromatic dicarboxylic acid,
b) at least one aliphatic dicarboxylic acid and
c) at least one aliphatic diol which has at least one branching point, a saturated cyclic partial structure and/or at least one ether group
as polymer base for gum bases, wherein the molar ratio of a) to b) is from 1:4 to 2:1.

2. The use according to claim 1, wherein the glass transition temperature T_{g} of the polyester is from 0 to -60°C.

3. The use according to either one of the preceding claims, wherein the aromatic dicarboxylic acid is selected from terephthalic acid, isophthalic acid and phthalic acid.

4. The use according to any one of the preceding claims, wherein the aliphatic dicarboxylic acid is selected from C₄-C₁₂ dicarboxylic acids.

5. The use according to claim 4, wherein the aliphatic C₄-C₁₂ dicarboxylic acid is selected from succinic acid, glutaric acid, adipic acid, pimelic acid, azelaic acid and sebacic acid.

6. The use according to any one of the preceding claims, wherein the aliphatic diol is selected from 2,2-dimethylpropane-1,3-diol, diethylene glycol, triethylene glycol, tetraethylene glycol, pentaethylene glycol, hexaethylene glycol and cyclohexanedimethanol.

7. The use according to any one of the preceding claims, wherein the polyester additionally comprises, as repeating unit, in condensed form,
d) at least one compound having at least three groups capable of ester formation.

8. The use according to claim 7, wherein the compound having at least three groups capable of ester formation is selected from tartaric acid, citric acid, malic acid, trimethylolpropane, trimethylolethane, pentaerythritol, polyethertriols, glycerol, trimesic acid, trimellitic acid, pyromellitic acid and hydroxyisophthalic acid.

9. The use according to either one of claims 7 and 8, wherein the polyester comprises the component d) in condensed form in an amount of from 0.1 to 5% by weight, based on the total weight of the polyester-forming components.

10. A chewing gum comprising a gum base which comprises at least one amorphous polyester as defined in any of the preceding claims and at least one further additive selected from resins, waxes, fats and oils, at least one sweetener and at least one flavoring.

## Revendications

1. Utilisation d'un polyester amorphe, qui contient sous forme condensée, comme unités répétitives,
a) au moins un acide dicarboxylique aromatique,
b) au moins un acide dicarboxylique aliphatique, et
c) au moins un diol aliphatique qui présente au moins un emplacement de ramification, une structure partielle cyclique saturée et/ou au moins un groupe éther,
comme base polymère pour des masses à mâcher, le rapport molaire entre a) et b) étant de 1/4 à 2/1.

2. Utilisation suivant la revendication 1, dans laquelle la température de transition vitreuse T_{g} du polyester est de 0 à -60°C.

3. Utilisation suivant l'une des revendications précédentes, dans laquelle l'acide dicarboxylique aromatique est choisi parmi de l'acide téréphtalique, de l'acide isophtalique et de l'acide phtalique.

4. Utilisation suivant l'une des revendications précédentes, dans laquelle l'acide dicarboxylique aliphatique est choisi parmi des acides dicarboxyliques en C₄-C₁₂.

5. Utilisation suivant la revendication 4, dans laquelle l'acide dicarboxylique aliphatique en C₄-C₁₂ est choisi parmi de l'acide succinique, de l'acide glutarique, de l'acide adipique, de l'acide pimélique, de l'acide azélaïque et de l'acide sébacique.

6. Utilisation suivant l'une des revendications précédentes, dans laquelle le diol aliphatique est choisi parmi du 2,2-diméthylpropane-1,3-diol, du diéthylèneglycol, du triéthylèneglycol, du tétraéthylèneglycol, du pentaéthylèneglycol, de l'hexaéthylèneglycol et du cyclohexanediméthanol.

7. Utilisation suivant l'une des revendications précédentes, dans laquelle le polyester contient en outre, sous forme condensée, comme unité répétitive,
d) au moins un composé comportant au moins trois groupes capables de formation d'ester.

8. Utilisation suivant la revendication 7, dans laquelle le composé comportant au moins trois groupes capables de formation d'ester est choisi parmi de l'acide tartrique, de l'acide citrique, de l'acide malique, du triméthylolpropane, du triméthyloléthane, de la pentaérythrite, des polyéthertriols, de la glycérine, de l'acide trimésique, de l'acide trimellitique, de l'acide pyromellitique et de l'acide hydroxyisophtalique.

9. Utilisation suivant la revendication 7 ou 8, dans laquelle le polyester contient sous forme condensée le composant d) en une quantité de 0,1 à 5 % en poids, par rapport au poids total des éléments formant le polyester.

10. Gomme à mâcher, contenant une masse à mâcher, qui comporte au moins un polyester amorphe tel que défini dans une des revendications précédentes, ainsi qu'au moins un autre additif choisi parmi des résines, des cires, des graisses et des huiles, au moins un agent édulcorant et au moins une substance sapide.
